# EUROPEAN PATENT APPLICATION

(11) **EP 4 810 592 A1**
(43) Date of publication of application: **23.09.2026**
(21) Application number: 24891020.0
(22) Date of filing: 22.07.2024
(51) Int. Cl.: C11B 9/00, A61K 8/34, A61K 8/35, A61Q 13/00

(54) **FRAGRANCE COMPOSITION**

(30) Priority: 15.11.2023 JP 2023194597
(71) Applicant: Kao Corporation, Tokyo 103-8210 (JP)
(72) Inventor: KOYAMA Shiori, Tokyo 103-8210 (JP); SAITO Akari, Tokyo 103-8210 (JP); MORI Hironori, Tokyo 103-8210 (JP); DEGUCHI Jun, Tokyo 103-8210 (JP); ARAI Tsubasa, Tokyo 103-8210 (JP)
(74) Representative: Hoffmann Eitle
(86) International application number: PCT/JP2024/026113
(87) International publication number: WO 2025/104976

(57) **Abstract**

A fragrance composition contains a compound represented by a general formula (I) and a compound represented by a general formula (II), wherein a mass ratio between the compound represented by the formula (I) and the compound represented by the formula (II) [compound represented by formula (I):compound represented by formula (II)] is 0.10:99.90 or more and 4.00:96.00 or less. [In the formulae (I) and (II), R^{I} and R³ are each independently an alkyl group having 1 or more and 5 or less carbon atoms, and R² and R⁴ are each independently a hydrogen atom or an alkyl group having 1 or more and 4 or less carbon atoms.]

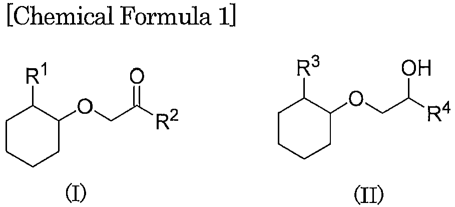

## Description

### Technical Field

The present invention relates to a fragrance composition containing a compound represented by a general formula (I) and a compound represented by a general formula (II).

### Background Art

Ketone compounds are known to exhibit useful activities in pharmaceuticals, fragrances, and pesticides.

JP H8-27056A (Patent Document 1) discloses that α-alkylcyclohexyloxyketones have a soft woody odor reminiscent of natural flowers and fruits and can, when added to a fragrance blend, alter its odor characteristics to ones extremely close to those of natural materials. 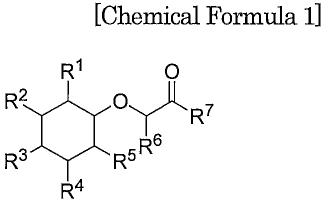 α-alkylcyclohexyloxyketones

JP H4-217937A (Patent Document 2) discloses that α-(alkylcyclohexyloxy)-β-alkanols have a woody and amber-like odor. 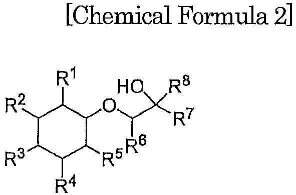 α-(alkylcyclohexyloxy)-β-alkanols

### Disclosure of Invention

The present invention provides a fragrance composition containing a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less.

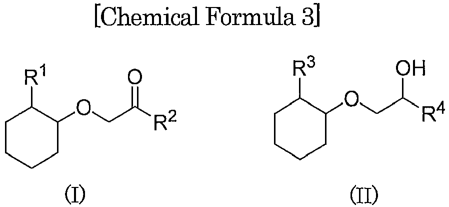

[In the formulae (I) and (II),
R¹ and R³ are each independently an alkyl group having 1 or more and 5 or less carbon atoms, and
R² and R⁴ are each independently a hydrogen atom or an alkyl group having 1 or more and 4 or less carbon atoms.]

### Description of the Invention

The present invention focuses on α-alkylcyclohexyloxyketones, which have not attracted attention conventionally, and aims to provide a novel fragrance composition.

The inventors of the present invention have surprisingly found that when α-alkylcyclohexyloxyketones are present at a specific ratio in a fragrance composition containing the α-alkylcyclohexyloxyketones and α-(alkylcyclohexyloxy)-8-alkanols, a fragrance composition having excellent balance, with orris-like sweetness and odor volume imparted to an amber-like and woody odor, can be obtained. Based on this finding, a fragrance composition of the present invention has been completed.

The present invention provides a fragrance composition containing a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less.

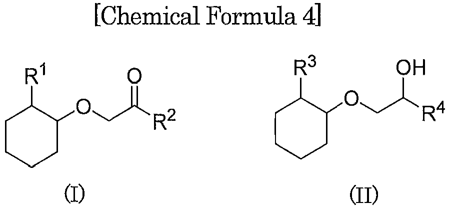

[In the formulae (I) and (II),
R¹ and R³ are each independently an alkyl group having 1 or more and 5 or less carbon atoms, and
R² and R⁴ are each independently a hydrogen atom or an alkyl group having 1 or more and 4 or less carbon atoms.]

The present invention also provides a method of using a composition as a fragrance-imparting component, the composition consisting of a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less.

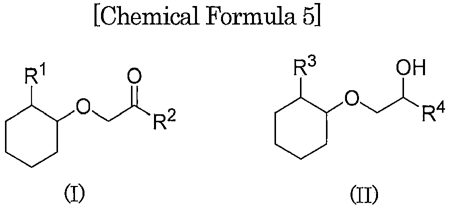

[In the formulae (I) and (II),
R¹ and R³ are each independently an alkyl group having 1 or more and 5 or less carbon atoms, and
R² and R⁴ are each independently a hydrogen atom or an alkyl group having 1 or more and 4 or less carbon atoms.]

The composition of the present invention, containing α-alkylcyclohexyloxyketones at a specific ratio, has the effect of providing excellent balance, with orris-like sweetness and odor volume imparted to its amber-like and woody odor, and is therefore useful as a fragrance composition.

As described above, the present invention provides a fragrance composition containing a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less.

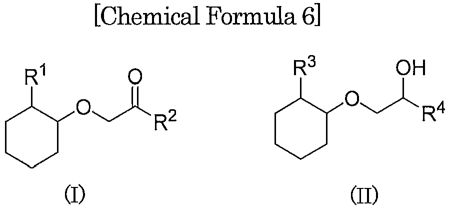

[In the formulae (I) and (II),
R¹ and R³ are each independently an alkyl group having 1 or more and 5 or less carbon atoms, and
R² and R⁴ are each independently a hydrogen atom or an alkyl group having 1 or more and 4 or less carbon atoms.]

From the viewpoint of imparting orris-like sweetness and volume, imparting persistence to the odor, and thereby providing a well-balanced fragrance composition, the content of the compound of the formula (II) in the fragrance composition is preferably 96.00 mass% or more, more preferably 97.00 mass% or more, and still more preferably 98.0 mass% or more; and is preferably 99.90 mass% or less, more preferably 99.80 mass% or less, and still more preferably 99.75 mass% or less. From the foregoing viewpoint, the content is preferably 96.00 mass% or more and 99.90 mass% or less, more preferably 97.00 mass% or more and 99.80 mass% or less, and still more preferably 98.00 mass% or more and 99.75 mass% or less.

The mass ratio of the compound represented by the formula (I) to the compound represented by the formula (II) [compound represented by formula (I):compound represented by formula (II)] is 0.10:99.90 or more, preferably 0.20:99.80 or more, and still more preferably 0.25:99.75 or more, from the viewpoint of imparting orris-like sweetness and volume, imparting persistence to the odor, and thereby providing a well-balanced fragrance composition. The mass ratio is 4.00:96.00 or less, preferably 3.00:97:00 or less, and still more preferably 2.00:98:00 or less, from the viewpoint of enhancing the amber-like and woody odor, imparting volume, and thereby providing a well-balanced fragrance composition.

From the foregoing viewpoints, the mass ratio of the compound represented by the formula (I) to the compound represented by the formula (II) [compound represented by formula (I):compound represented by formula (II)] is 0.10:99.90 or more and 4.00:96.00 or less, preferably 0.20:99.80 or more and 3.00:97:00 or less, and still more preferably 0.25:99.75 or more and 2.00:98:00 or less.

Note that the mass ratio of the compound represented by the formula (I) to the compound represented by the formula (II) [compound represented by formula (I):compound represented by formula (II)] being 0.10:99.90 or more means that the mass ratio of compound represented by formula (I)/compound represented by formula (II) is 0.10/99.90 or more, and the mass ratio of the compound represented by the formula (I) to the compound represented by the formula (II) [compound represented by formula (I):compound represented by formula (II)] being 4.00:96.00 or less means that the mass ratio of compound represented by formula (I)/compound represented by formula (II) is 4.00/96.00 or less.

In the present specification, examples of the alkyl group having 1 or more and 5 or less carbon atoms in R¹ and R³ include straight-chain or branched alkyl groups such as methyl, ethyl, n-propyl, i-propyl, n-butyl, i-butyl, sec-butyl, t-butyl, n-pentyl, i-pentyl, sec-pentyl, t-pentyl, and 2-methylbutyl groups. The alkyl group having 1 or more and 5 or less carbon atoms is preferably an alkyl group having 1 or more and 4 or less carbon atoms, more preferably an alkyl group having 3 or more and 4 or less carbon atoms, and still more preferably a t-butyl group.

In the present specification, examples of the alkyl group having 1 or more and 4 or less carbon atoms in R² and R⁴ include straight-chain or branched alkyl groups such as methyl, ethyl, n-propyl, i-propyl, n-butyl, i-butyl, sec-butyl, and t-butyl groups. The alkyl group having 1 or more and 4 or less carbon atoms is preferably an alkyl group having 1 or more and 3 or less carbon atoms, more preferably an alkyl group having 2 or more and 3 or less carbon atoms, and still more preferably an ethyl group, so as to impart orris-like sweetness and odor volume to the amber-like and woody odor, thereby allowing the fragrance composition of the present invention to have excellent balance.

In the formula (I), R¹ is preferably an alkyl group having 1 or more and 4 or less carbon atoms, more preferably an alkyl group having 3 or more and 4 or less carbon atoms, and still more preferably a t-butyl group, so as to impart orris-like sweetness and odor volume to the amber-like and woody odor, thereby allowing the fragrance composition of the present invention to have excellent balance.

In the formula (I), R² is preferably an alkyl group having 1 or more and 3 or less carbon atoms, more preferably an alkyl group having 2 or more and 3 or less carbon atoms, and still more preferably an ethyl group, so as to impart orris-like sweetness and odor volume to the amber-like and woody odor, thereby allowing the fragrance composition of the present invention to have excellent balance.

As the compound represented by the formula (I), for example, compounds having the combinations of R¹ and R² shown in Table 1 below are preferably employed so as to impart orris-like sweetness and odor volume to the amber-like and woody odor, thereby allowing the fragrance composition of the present invention to have excellent balance. Of these, a compound in which R¹ is a t-butyl group and R² is an ethyl group is more preferable.

**[Table 1]**

| | R¹ | R² |
|---|---|---|
| Combination I-1 | Alkyl group having 1 or more and 5 or less carbon atoms | Hydrogen atom or alkyl group having 1 or more and 4 or less carbon atoms |
| Combination I-2 | Alkyl group having 1 or more and 5 or less carbon atoms | Alkyl group having 1 or more and 3 or less carbon atoms |
| Combination I-3 | Alkyl group having 1 or more and 5 or less carbon atoms | Alkyl group having 2 or more and 3 or less carbon atoms |
| Combination I-4 | Alkyl group having 1 or more and 5 or less carbon atoms | Ethyl group |
| Combination I-5 | Alkyl group having 1 or more and 4 or less carbon atoms | Hydrogen atom or alkyl group having 1 or more and 4 or less carbon atoms |
| Combination I-6 | Alkyl group having 1 or more and 4 or less carbon atoms | Alkyl group having 1 or more and 3 or less carbon atoms |
| Combination I-7 | Alkyl group having 1 or more and 4 or less carbon atoms | Alkyl group having 2 or more and 3 or less carbon atoms |
| Combination I-8 | Alkyl group having 1 or more and 4 or less carbon atoms | Ethyl group |
| Combination I-9 | Alkyl group having 3 or more and 4 or less carbon atoms | Hydrogen atom or alkyl group having 1 or more and 4 or less carbon atoms |
| Combination I-10 | Alkyl group having 3 or more and 4 or less carbon atoms | Alkyl group having 1 or more and 3 or less carbon atoms |
| Combination I-11 | Alkyl group having 3 or more and 4 or less carbon atoms | Alkyl group having 2 or more and 3 or less carbon atoms |
| Combination I-12 | Alkyl group having 3 or more and 4 or less carbon atoms | Ethyl group |
| Combination I-13 | t-Butyl group | Hydrogen atom or alkyl group having 1 or more and 4 or less carbon atoms |
| Combination I-14 | t-Butyl group | Alkyl group having 1 or more and 3 or less carbon atoms |
| Combination I-15 | t-Butyl group | Alkyl group having 2 or more and 3 or less carbon atoms |
| Combination I-16 | t-Butyl group | Ethyl group |

As the compound represented by the formula (I), for example, the following compound is preferably employed so as to impart orris-like sweetness and odor volume to the amber-like and woody odor, thereby allowing the fragrance composition of the present invention to have excellent balance.

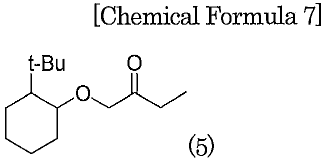

The compound represented by the formula (I) may be a mixture of the following cis isomer and trans isomer, or may be either one thereof.

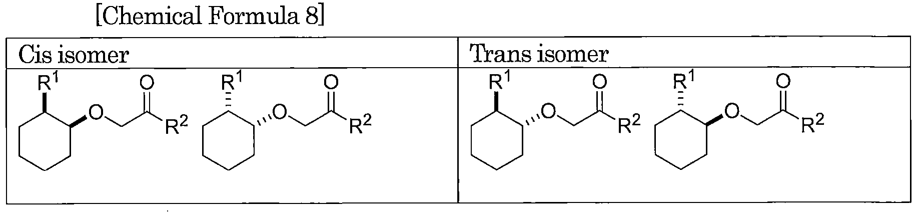

In the formula (II), R³ is preferably an alkyl group having 1 or more and 4 or less carbon atoms, more preferably an alkyl group having 3 or more and 4 or less carbon atoms, and still more preferably a t-butyl group, so as to impart orris-like sweetness and odor volume to the amber-like and woody odor, thereby allowing the fragrance composition of the present invention to have excellent balance.

In the formula (II), R⁴ is preferably an alkyl group having 1 or more and 3 or less carbon atoms, more preferably an alkyl group having 2 or more and 3 or less carbon atoms, and still more preferably an ethyl group, so as to impart orris-like sweetness and odor volume to the amber-like and woody odor, thereby allowing the fragrance composition of the present invention to have excellent balance.

As the compound represented by the formula (II), for example, compounds having the combinations of R³ and R⁴ shown in Table 2 below are preferably employed so as to impart orris-like sweetness and odor volume to the amber-like and woody odor, thereby allowing the fragrance composition of the present invention to have excellent balance. Of these, a compound in which R³ is a t-butyl group and R⁴ is an ethyl group is more preferable.

**[Table 2]**

| | R³ | R⁴ |
|---|---|---|
| Combination II-1 | Alkyl group having 1 or more and 5 or less carbon atoms | Hydrogen atom or alkyl group having 1 or more and 4 or less carbon atoms |
| Combination II-2 | Alkyl group having 1 or more and 5 or less carbon atoms | Alkyl group having 1 or more and 3 or less carbon atoms |
| Combination II-3 | Alkyl group having 1 or more and 5 or less carbon atoms | Alkyl group having 2 or more and 3 or less carbon atoms |
| Combination II-4 | Alkyl group having 1 or more and 5 or less carbon atoms | Ethyl group |
| Combination II-5 | Alkyl group having 1 or more and 4 or less carbon atoms | Hydrogen atom or alkyl group having 1 or more and 4 or less carbon atoms |
| Combination II-6 | Alkyl group having 1 or more and 4 or less carbon atoms | Alkyl group having 1 or more and 3 or less carbon atoms |
| Combination II-7 | Alkyl group having 1 or more and 4 or less carbon atoms | Alkyl group having 2 or more and 3 or less carbon atoms |
| Combination II-8 | Alkyl group having 1 or more and 4 or less carbon atoms | Ethyl group |
| Combination II-9 | Alkyl group having 3 or more and 4 or less carbon atoms | Hydrogen atom or alkyl group having 1 or more and 4 or less carbon atoms |
| Combination II-10 | Alkyl group having 3 or more and 4 or less carbon atoms | Alkyl group having 1 or more and 3 or less carbon atoms |
| Combination II-11 | Alkyl group having 3 or more and 4 or less carbon atoms | Alkyl group having 2 or more and 3 or less carbon atoms |
| Combination II-12 | Alkyl group having 3 or more and 4 or less carbon atoms | Ethyl group |
| Combination 11-13 | t-Butyl group | Hydrogen atom or alkyl group having 1 or more and 4 or less carbon atoms |
| Combination II-14 | t-Butyl group | Alkyl group having 1 or more and 3 or less carbon atoms |
| Combination II-15 | t-Butyl group | Alkyl group having 2 or more and 3 or less carbon atoms |
| Combination II-16 | t-Butyl group | Ethyl group |

As examples of the compound represented by the formula (II), the following compound is preferably employed so as to impart orris-like sweetness and odor volume to the amber-like and woody odor, thereby allowing the fragrance composition of the present invention to have excellent balance.

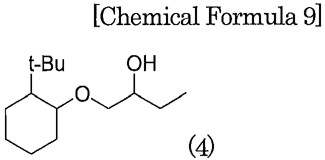

The compound represented by the formula (II) may be a mixture of the following cis isomer and trans isomer, or may be either one thereof.

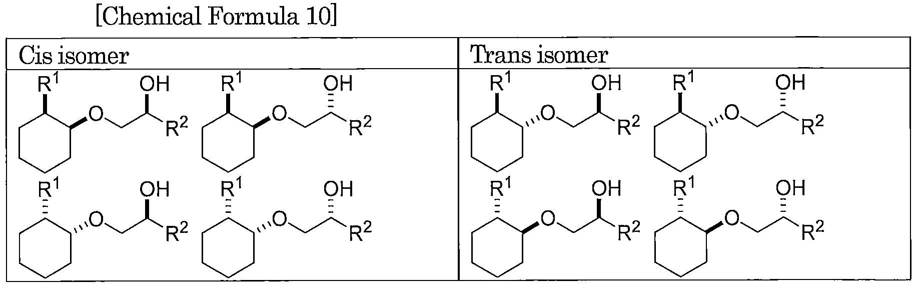

In the fragrance composition of the present invention, it is preferable that R¹ and R³ are t-butyl groups so as to impart orris-like sweetness and odor volume to the amber-like and woody odor, thereby allowing the fragrance composition of the present invention to have excellent balance.

In the fragrance composition of the present invention, it is preferable that R² and R⁴ are independently a methyl group or an ethyl group so as to impart orris-like sweetness and odor volume to the amber-like and woody odor, thereby allowing the fragrance composition of the present invention to have excellent balance.

In the fragrance composition of the present invention, it is more preferable that R¹ and R³ are t-butyl groups and R² and R⁴ are independently a methyl group or an ethyl group so as to impart orris-like sweetness and odor volume to the amber-like and woody odor, thereby allowing the fragrance composition of the present invention to have excellent balance.

In the fragrance composition of the present invention, examples of the combination of the compound represented by the formula (I) and the compound represented by the formula (II) include a combination of one or more selected from the group consisting of Combinations I-1, I-2, I-3, I-4, I-5, I-6, I-7, I-8, I-9, I-10, I-11, I-12, I-13, I-14, I-15, and I-16 shown in Table 1 and one or more selected from the group consisting of Combinations II-1, II-2, II-3, II-4, II-5, II-6, II-7, II-8, II-9, II-10, II-11, II-12, II-13, II-14, II-15, and II-16 shown in Table 2. As the combination of the compound represented by the formula (I) and the compound represented by the formula (II), a combination of Combination I-16 and Combination II-16 is more preferably employed so as to impart orris-like sweetness and odor volume to the amber-like and woody odor, thereby allowing the fragrance composition of the present invention to have excellent balance. In other words, it is preferable that R¹ is a t-butyl group, R² is an ethyl group, R³ is a t-butyl group, and R⁴ is an ethyl group.

The compound represented by the formula (I) can be produced using a method described in JP H8-27056A, for example.

The compound represented by the formula (I) can also be produced using the following method, for example.

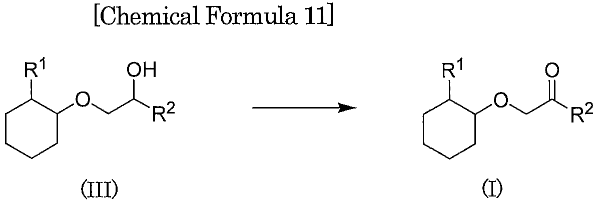

In the formulae (I) and (III),
R¹ is an alkyl group having 1 or more and 5 or less carbon atoms, and
R² is a hydrogen atom or an alkyl group having 1 or more and 4 or less carbon atoms.

The method includes a step of oxidizing a compound of the formula (III) to thereby obtain a compound of the formula (I). The step may be performed by, for example, oxidizing the compound of the formula (III) using an oxidizing agent (such as, for example, Dess-Martine periodinane) to obtain the compound of the formula (I). The thus-obtained compound of the formula (I) can be separated and purified by distillation or column chromatography.

The compound represented by the formula (II) can be produced using a method described in JP H4-217937A, for example.

In addition, it is preferable that the fragrance composition of the present invention further contains at least one component that is other than the compounds represented by the formulae (I) and (II) and is selected from alcohols, phenols, esters, carbonates, aldehydes, ketones, acetals, ethers, lactones, and nitriles so as to impart orris-like sweetness and odor volume to the amber-like and woody odor, thereby allowing the fragrance composition of the present invention to have excellent balance.

In the present specification, each such fragrance "class" refers to either a single compound or a mixture of two or more compounds.

Examples of the alcohols include aliphatic alcohols, terpene alcohols, aromatic alcohols, and other alcohols. Of these, aliphatic alcohols are preferable.

Examples of the terpene alcohols include linalool, ethyl linalool, citronellol, hydroxycitronellol, geraniol, tetrahydrogeraniol, nerol, terpineol, α-terpineol, dihydromyrcenol, farnesol, nerolidol, cedrol, menthol, and borneol.

Examples of the aromatic alcohols include phenylethyl alcohol, benzyl alcohol, dimethyl benzyl carbinol, phenylethyl dimethyl carbinol, and phenyl hexanol.

Examples of the aliphatic alcohols include cis-3-hexenol, 1-(2,2,6-trimethylcyclohexyl)-3-hexanol, Sandalmysore Core (trade name, manufactured by Kao Corporation; 2-methyl-4-(2,2,3-trimethyl-3-cyclopenten-1-yl)-2-buten-1-ol), Magnol (trade name, manufactured by Kao Corporation; a mixture containing ethyl norbornyl cyclohexanol as a main component), Undecavertol (trade name, manufactured by Givaudan; 4-methyl-3-decen-5-ol), isobornyl cyclohexanol, and Terpirosa (trade name, manufactured by Kao Corporation; 3,6-dimethyl-2-heptanol).

Examples of other alcohols include glycols such as dipropylene glycol, Florosa (trade name, manufactured by Givaudan; chemical name: 4-methyl-2-(2-methylpropyl)tetrahydro-2H-4-pyranol), maltol, and ethyl maltol.

Examples of the hydrocarbons include limonene, α-pinene, β-pinene, terpinene, cedrene, longifolene, and valencene.

Examples of the phenols include guaiacol, eugenol, dihydroeugenol, isoeugenol, thymol, para-cresol, vanillin, and ethyl vanillin.

Examples of the esters include aliphatic carboxylic acid esters, aromatic carboxylic acid esters, and other carboxylic acid esters.

Examples of aliphatic carboxylic acids that form the aliphatic carboxylic acid esters include straight-chain and branched carboxylic acids having 1 to 18 carbon atoms. Of these, carboxylic acids having 1 to 6 carbon atoms, such as formic acid, acetic acid, and propionic acid, are preferable, and acetic acid is more preferable. Examples of aromatic carboxylic acids that form the aromatic carboxylic acid esters include benzoic acid, anisic acid, phenylacetic acid, cinnamic acid, salicylic acid, and anthranilic acid. Examples of alcohols that form the aliphatic and aromatic esters include straight-chain and branched aliphatic alcohols having 1 to 5 carbon atoms and the alcohols described above as examples of the fragrance components.

Examples of formic acid esters include linalyl formate, citronellyl formate, and geranyl formate.

Examples of acetic acid esters include ethyl acetate, isoamyl acetate (isopentyl acetate), benzyl acetate, hexyl acetate, phenyl acetate, p-cresyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, phenylethyl acetate, styralyl acetate, cinnamyl acetate, dimethylbenzylcarbinyl acetate, 3-pentyltetrahydropyran-4-yl acetate, prenyl acetate, geranyl acetate, and methylphenylcarbinyl acetate; and examples of phenylacetic acid esters include phenylethyl phenylacetate and p-cresyl phenylacetate.

Examples of propionic acid esters include ethyl propionate, citronellyl propionate, tricyclodecenyl propionate, benzyl propionate, styralyl propionate, and Helvetolide (trade name, manufactured by Firmenich; 2-[1-(3,3-dimethylcyclohexyl)ethoxy]-2-methylpropyl)propionate).

Examples of butyric acid esters include citronellyl butyrate, dimethylbenzylcarbinyl n-butyrate, isoamyl n-butyrate, and n-amyl n-butyrate; and examples of isobutyric acid esters include tricyclodecenyl isobutyrate and phenoxyethyl isobutyrate.

Examples of valeric acid esters include methyl valerate, ethyl valerate, butyl valerate, amyl valerate, benzyl valerate, and phenylethyl valerate; and examples of hexanoic acid esters include methyl hexanoate, ethyl hexanoate, allyl hexanoate, linalyl hexanoate, and citronellyl hexanoate.

Examples of heptanoic acid esters include methyl heptanoate and allyl heptanoate.

Examples of nonenoic acid esters include methyl 2-nonenoate, ethyl 2-nonenoate, and ethyl 3-nonenoate.

Examples of benzoic acid esters include methyl benzoate, benzyl benzoate, and 3,6-dimethyl benzoate.

Examples of cinnamic acid esters include methyl cinnamate and benzyl cinnamate.

Examples of salicylic acid esters include methyl salicylate, n-hexyl salicylate, cis-3-hexenyl salicylate, cyclohexyl salicylate, and benzyl salicylate (benzyl 2-hydroxybenzoate).

Examples of brassylic acid esters include ethylene brassylate.

Examples of tiglic acid esters include geranyl tiglate, 1-hexyl tiglate, and cis-3-hexenyl tiglate.

Examples of jasmonic acid esters include methyl jasmonate; and examples of dihydrojasmonic acid esters include MDJ (trade name, manufactured by Kao Corporation; methyl dihydrojasmonate, methyl(2-pentyl-3-oxocyclopentylacetate).

Examples of glycidic acid esters include methyl 2,4-dihydroxy-ethyl methylphenyl glycidate and 4-methylphenyl ethyl glycidate.

Examples of anthranilic acid esters include methyl anthranilate, ethyl anthranilate, and dimethyl anthranilate.

Examples of glycolic acid esters include allylcyclohexylglycolate.

Furthermore, examples of other esters include Ethyl Safranate (trade name, manufactured by Givaudan; ethyl dihydrocyclogeranate), Poirenate (trade name, manufactured by Kao Corporation; ethyl-2-cyclohexyl propionate), Fruitate (trade name, manufactured by Kao Corporation; ethyl tricyclo[5.2.1.0^{2.6}]decane-2-carboxylate), ethyl acetoacetate, Fructone (trade name, manufactured by IFF; ethyl 2-(2-methyl-1,3-dioxolan-2-yl)acetate), Manzanate (trade name, manufactured by Givaudan; ethyl 2-methylpentanoate), ethyl 2-methylbutyrate, allylcyclohexylpropionate, and allyl-2-pentyloxyglycolate.

Examples of the carbonates include Liffarome (trade name, manufactured by IFF; cis-3-hexenyl methyl carbonate), Jasmacyclat (trade name, manufactured by Kao Corporation; methyl-cyclooctyl carbonate), and Floramat (trade name, manufactured by Kao Corporation; ethyl-2-t-butylcyclohexyl carbonate).

Examples of the aldehydes include n-octanal, n-nonanal, n-decanal, n-dodecanal, 2-methylundecanal, 10-undecenal, citronellal, citral, hydroxycitronellal, Triplal (trade name, manufactured by IFF; 2,4-dimethyl-3-cyclohexene-1-carboxyaldehyde), Cyclovertal (trade name, manufactured by Kao Corporation; dimethyl-3-cyclohexenyl-1-carboxaldehyde), benzaldehyde, phenylacetaldehyde, phenylpropyl aldehyde, cinnamic aldehyde, dimethyltetrahydrobenzaldehyde, Bourgeonal (trade name, manufactured by Givaudan; 3-(4-tert-butylphenyl)propanal), Lyral (trade name, manufactured by IFF; hydroxy myrac aldehyde), Pollenal II (trade name, manufactured by Kao Corporation; 2-cyclohexyl propanal), Lilial (trade name, manufactured by Givaudan; p-tert-butyl-α-methylhydrocinnamic aldehyde), p-isopropyl-α-methylhydrocinnamic aldehyde, Floralozone (trade name, manufactured by IFF; 3-(o-(and p-)ethylphenyl)-2,2-dimethylpropionaldehyde), α-amyl cinnamic aldehyde, α-hexyl cinnamic aldehyde, Heliotropin (trade name, manufactured by Takasago International Corporation; 3,4-methylenedioxybenzaldehyde), Helional (trade name, manufactured by IFF; alpha-methyl-1,3-benzodioxole-5-propanal), Canthoxal (trade name, manufactured by IFF; 2-methyl-3-(para-methoxyphenyl)propanal), and Aldehyde C-6 (trade name, manufactured by Kao Corporation; n-hexanal).

Examples of the ketones include methylheptenone, dimethyloctenone, 3-octanone, hexylcyclopentanone, dihydrojasmone, Veloutone (trade name, manufactured by Firmenich; 2,2,5-trimethyl-5-pentylcyclopentanone), Nectaryl (trade name, manufactured by Givaudan; 2-(2-(4-methyl-3-cyclohexen-1-yl)propyl)cyclopentanone), ionone, β-ionone, methyl ionone, methyl ionone-G, γ-methyl ionone, damascone, α-damascone, β-damascone, δ-damascone, Isodamascone (trade name, manufactured by Symrise; 1-(2,4,4-trimethyl-2-cyclohexyl)-trans-2-butanone), damascenone, Dynascone (trade name, manufactured by Firmenich; 1-(5,5-dimethyl-1-cyclohexen-1-yl)-4-penten-1-one), irone, Cashmeran (trade name, manufactured by IFF; 1,2,3,5,6,7-hexahydro-1,1,2,3,3-pentamethyl-4H-inden-4-one), Iso E Super (trade name, manufactured by IFF; 1-(1,2,3,4,5,6,7,8-octahydro-2,3,8,8-tetramethyl-2-naphthalenyl)-ethan-1-one), Calone (trade name, manufactured by Firmenich; 7-methyl-3,4-dihydro-2H-benzodioxepin-3-one), carvone, menthone, acetyl cedrene, isolongifolanone, nootkatone, benzylacetone, raspberry ketone, benzophenone, Tonalide (trade name, manufactured by PFW, 6-acetyl-1,1,2,4,4,7-hexamethyltetrahydronaphthalene), β-methylnaphthyl ketone, ethyl maltol, camphor, muscone, Muscenone (trade name, manufactured by Firmenich; 3-methyl-5-cyclopentadecen-1-one), civetone, Glovanon (trade name, manufactured by Symrise; 8-cyclohexadecenone), methylnonyl ketone, cis-jasmone, para-amylcyclohexanone (4-pentyl cyclohexanone), and Tonalide (trade name, manufactured by PFW Aroma Chemicals; 1-(3,5,5,6,8,8-hexamethyl-5,6,7,8-tetrahydronaphthalen-2-ylethan-1-one). Of these, ionone, damascenone, Iso E Super, or γ-methyl ionone is preferable from the viewpoint of enhancing floral sweetness when blended with the fragrance composition of the present invention.

Examples of the acetals include acetaldehyde ethylphenylpropyl acetal, citral diethyl acetal, phenylacetaldehyde glyceryl acetal, ethyl acetoacetate ethylene glycol acetal, Boisambrene Forte (trade name, manufactured by Kao Corporation; ethoxymethyl cyclododecyl ether), Troenan (trade name, manufactured by Kao Corporation; 5-methyl-5-propyl-2-(1-methylbutyl)-1,3-dioxane), Floropal (trade name, manufactured by Symrise; 2,4,6-trimethyl-4-phenyl-1,3-dioxane), and Magnolan (trade name, manufactured by Symrise; 2,4-dimethyl-4,4a,5,9b-tetrahydroindeno[1,2-d][1,3]dioxane).

Examples of the ethers include cedryl methyl ether, estragole, anethole, β-naphthyl methyl ether, β-naphthyl ethyl ether, limonene oxide, rose oxide, nerol oxide, 1,8-cineole, rosefuran, Ambroxan (trade name, manufactured by Kao Corporation; [3aR-(3aα,5aβ,9aα,9bβ)]dodecahydro-3a,6,6,9a-tetramethylnaphtho[2,1-b]furan), Herbavert (trade name, manufactured by Kao Corporation; 3,3,5-trimethylcyclohexyl ethyl ether), Galaxolide (trade name, manufactured by IFF; hexamethylhexahydrocyclopentabenzopyran), phenylacetaldehyde dimethylacetal, and methyl isoeugenol.

Examples of the carboxylic acids include benzoic acid, phenylacetic acid, cinnamic acid, hydrocinnamic acid, butyric acid, and 2-hexenoic acid.

Examples of the lactones include ambrettolide, γ-decalactone, δ-decalactone, γ-valerolactone, γ-nonalactone, γ-undecalactone, 6-hexalactone, γ-jasmolactone, whisky lactone, coumarin, cyclopentadecanolide, cyclohexadecanolide, 11-oxahexadecanolide, and butylidenephthalide.

Examples of the nitriles include tridecene-2-nitrile, geranyl nitrile, citronellyl nitrile, and dodecanenitrile.

It is also preferable that the fragrance composition of the present invention further contains natural essential oils or natural extracts so as to impart orris-like sweetness and odor volume to the amber-like and woody odor, thereby allowing the fragrance composition of the present invention to have excellent balance.

Examples of the natural essential oils and the natural extracts include oils, concretes, absolutes, resinoids, oleoresins, tinctures, infusions, and balsams of natural raw materials, such as orange, lemon, lime, bergamot, vanilla, mandarin, peppermint, spearmint, lavender, galbanum, chamomile, rosemary, eucalyptus, sage, basil, rose, rockrose, geranium, jasmine, ylang-ylang, anise, clove, ginger, nutmeg, cardamom, cedar, Japanese cypress, vetiver, patchouli, hay, lemongrass, labdanum, grapefruit, elemi oils, and bergamot oils.

The total content of the compound represented by the formula (I) and the compound represented by the formula (II) in the fragrance composition of the present invention can be selected as appropriate according to, for example, the type of fragrance blend and the type and intensity of intended aromatic odor. However, from the viewpoint of imparting an aromatic odor having excellent balance in which orris-like sweetness and odor volume are imparted to the amber-like and woody odor, the total content of these compounds in a composition containing the fragrance composition of the present invention is preferably 0.0001 mass% or more, more preferably 0.001 mass% or more, still more preferably 0.1 mass% or more, and yet still more preferably 1 mass% or more; is preferably 99.99 mass% or less, more preferably 80 mass% or less, still more preferably 60 mass% or less, and yet still more preferably 50 mass% or less; and is preferably 0.0001 mass% or more and 99.99 mass% or less, more preferably 0.001 mass% or more and 80 mass% or less, still more preferably 0.1 mass% or more and 60 mass% or less, and yet still more preferably 1 mass% or more and 50 mass% or less.

The fragrance composition of the present invention may be used in combination with an oil that serves as a base and itself is odorless. Such an oil allows fragrance components to be uniformly mixed together and to be easily added to a product, thereby allowing an odor with a moderate intensity to be more easily imparted to the product. Examples of the oil include polyhydric alcohols such as ethylene glycol, propylene glycol, butylene glycol, and dipropylene glycol; esters such as isopropyl myristate, dibutyl adipate, and diethyl sebacate; hydrocarbons such as liquid paraffin and squalane; and surfactants such as polyoxyethylene alkyl ethers and sorbitan fatty acid esters.

Of these, polyhydric alcohols and esters are preferable as the oil from the viewpoint of improving solubility of all the fragrance components, and dipropylene glycol and isopropyl myristate are more preferable. The content of such an oil in the fragrance composition is preferably 0.01 mass% or more, more preferably 1 mass% or more, and still more preferably 5 mass% or more; and is preferably 95 mass% or less, more preferably 90 mass% or less, and still more preferably 80 mass% or less.

The present invention further provides a cleaning composition containing the fragrance composition of the present invention, a cosmetic containing the fragrance composition of the present invention, and a fiber treating composition containing the fragrance composition of the present invention.

The cleaning composition of the present invention is preferably a body cleaning composition, a cleaning composition for clothing, or a cleaning composition for hard surfaces, more preferably a body cleaning composition or a cleaning composition for clothing, and still more preferably a cleaning composition for clothing.

The body cleaning composition is, for example, a skin cleaning composition, a hair cleaning composition, or a soap composition, and is preferably a skin cleaning composition.

The cleaning composition for hard surfaces is, for example, an all-purpose cleaner or a cleaning composition for tableware.

The fiber treating composition of the present invention is preferably a fabric softener composition.

The cosmetic of the present invention, excluding cleaning compositions, is preferably a perfume, a milky lotion, a skin lotion, or a sunscreen, and more preferably a perfume.

The cleaning composition of the present invention preferably contains an anionic surfactant in addition to the fragrance composition of the present invention, and may further contain a nonionic surfactant, a pH adjuster, a viscosity modifier, a solvent, an oil, a preservative, water, etc.

The fiber treating composition of the present invention preferably contains a cationic surfactant in addition to the fragrance composition of the present invention, and may further contain a pH adjuster, a solvent, an oil, a preservative, water, etc.

The perfume of the present invention may contain a solvent, water, or the like, in addition to the fragrance composition of the present invention.

The present invention also provides a method of using a composition as a fragrance-imparting component, the composition consisting of a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less.

The preferred mass ratio of the compound represented by the formula (I) to the compound represented by the formula (II) [compound represented by formula (I):compound represented by formula (II)] is as described above.

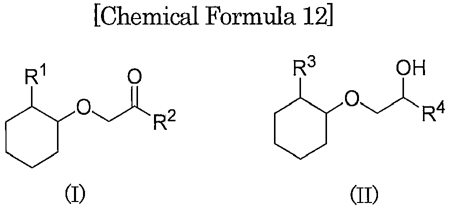

[In the formulae (I) and (II),
R¹ and R³ are each independently an alkyl group having 1 or more and 5 or less carbon atoms, and
R² and R⁴ are each independently a hydrogen atom or an alkyl group having 1 or more and 4 or less carbon atoms.]

The preferred groups for R¹, R², R³, and R⁴ are as described above.

The method of using the above-described composition of the present invention as a fragrance-imparting component is specifically a method of using the same as a fragrance-imparting component in a fragrance composition, a cleaning composition, a cosmetic, or a fiber treating composition. The cleaning composition is preferably a body cleaning composition, a cleaning composition for clothing, or a cleaning composition for hard surfaces, more preferably a body cleaning composition or a cleaning composition for clothing, and still more preferably a cleaning composition for clothing. The body cleaning composition is, for example, a skin cleaning composition or a hair cleaning composition, and is preferably a skin cleaning composition. The cleaning composition for hard surfaces is, for example, an all-purpose cleaner or a cleaning composition for tableware. The cosmetic is preferably a perfume. The fiber treating composition is preferably a fabric softener composition.

In the above-described method of use, the above-described composition is used in an amount of preferably 0.0001 mass% or more and more preferably 0.001 mass% or more, and preferably 30 mass% or less, more preferably 20 mass% or less, still more preferably 10 mass% or less, 1 mass% or less, and yet still more preferably 0.1 mass% or less, in the cleaning composition, the cosmetic, or the fabric softener composition. When used in such an amount, the above-described composition, serving as a fragrance material, can create a new impression by providing excellent balance in which orris-like sweetness and odor volume are imparted to the amber-like and woody odor.

In the above-described method of use, the above-described composition may be used in combination with an oil that itself is odorless. The oil is the same as that described above in connection with the fragrance composition. In addition, in the above-described method of use, the above-described composition may be used in combination with, as an additional fragrance, a commonly used other fragrance component or a fragrance blend having a desired composition. Such additional fragrances are the same as those described above in connection with the fragrance composition.

The present invention includes the following embodiments.
<1> A fragrance composition containing a compound represented by a general formula (I) and a compound represented by a general formula (II),
   wherein a mass ratio of the compound represented by the formula (I) to the compound represented by the formula (II) [compound represented by formula (I):compound represented by formula (II)] is 0.10:99.90 or more and 4.00:96.00 or less. 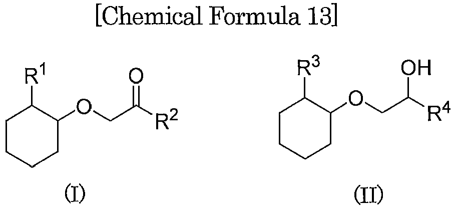 [In the formulae (I) and (II),
   R¹ and R³ are each independently an alkyl group having 1 or more and 5 or less carbon atoms, and
   R² and R⁴ are each independently a hydrogen atom or an alkyl group having 1 or more and 4 or less carbon atoms.]
<2> The fragrance composition according to <1>, wherein R¹ and R³ are t-butyl groups.
<3> The fragrance composition according to <1> or <2>, wherein R² and R⁴ are independently a methyl group or an ethyl group.
<4> The fragrance composition according to any one of <1> to <3>, wherein R¹ and R³ are t-butyl groups, and R² and R⁴ are ethyl groups.
<5> The fragrance composition according to any one of <1> to <4>, wherein the mass ratio of the compound represented by the formula (I) to the compound represented by the formula (II) [compound represented by formula (I):compound represented by formula (II)] is 0.20:99.80 or more and 3.00:97:00 or less.
<6> The fragrance composition according to any one of <1> to <5>, wherein the mass ratio of the compound represented by the formula (I) to the compound represented by the formula (II) [compound represented by formula (I):compound represented by formula (II)] is 0.25:99.75 or more and 2.00:98:00 or less.
<7> The fragrance composition according to any one of <1> to <6>, wherein a total content of the compound represented by the formula (I) and the compound represented by the formula (II) in the fragrance composition is 0.0001 mass% or more and 99.99 mass% or less.
<8> The fragrance composition according to any one of <1> to <7>, wherein a total content of the compound represented by the formula (I) and the compound represented by the formula (II) in the fragrance composition is 0.001 mass% or more and 80 mass% or less.
<9> The fragrance composition according to any one of <1> to <8>, wherein a total content of the compound represented by the formula (I) and the compound represented by the formula (II) in the fragrance composition is more preferably 0.1 mass% or more and 60 mass% or less.
<10> The fragrance composition according to any one of <1> to <9>, wherein a total content of the compound represented by the formula (I) and the compound represented by the formula (II) in the fragrance composition is 1 mass% or more and 50 mass% or less.
<11> The fragrance composition according to any one of <1> to <10>, further containing at least one component that is other than the compounds represented by the formulae (I) and (II) and is selected from alcohols, phenols, esters, carbonates, aldehydes, ketones, acetals, ethers, lactones, and nitriles.
<12> The fragrance composition according to any one of <1> to <11>, further containing a natural essential oil or a natural extract that is other than the compounds represented by the formulae (I) and (II).
<13> A cosmetic containing the fragrance composition according to any one of <1> to <12>.
<14> A cleaning composition containing the fragrance composition according to any one of <1> to <12>.
<15> A fiber treating agent composition containing the fragrance composition according to any one of <1> to <12>.
<16> A method of using a composition as a fragrance-imparting component, the composition consisting of a compound represented by a general formula (I) and a compound represented by a general formula (II),
   wherein a mass ratio of the compound represented by the formula (I) to the compound represented by the formula (II) [compound represented by formula (I):compound represented by formula (II)] is 0.10:99.90 or more and 4.00:96.00 or less.

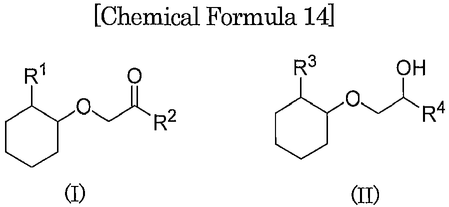

[In the formulae (I) and (II),
R¹ and R³ are each independently an alkyl group having 1 or more and 5 or less carbon atoms, and
R² and R⁴ are each independently a hydrogen atom or an alkyl group having 1 or more and 4 or less carbon atoms.]

In the following examples, "%" is "mass%", unless otherwise specified. The mass of a catalyst refers to the mass of the catalyst in a dry state.

### <Apparatus and Analytical Conditions for Gas Chromatography>

GC apparatus: 7890B, manufactured by Agilent Technologies, Inc., with hydrogen flame ionization detector
Column: For analysis of the yield, DB-1 (capillary column, 100% dimethylpolysiloxane, inner diameter: 0.25 mm, length: 30 m, film thickness: 0.25 pm, manufactured by Agilent Technologies, Inc.) was used.

For analysis of cis and trans isomers, DB-WAX (capillary column, polyethylene glycol, inner diameter: 0.25 mm, length: 30 m, film thickness: 0.25 pm, manufactured by Agilent Technologies, Inc.) was used.
Carrier gas: He, 1.5 mL/min
Injection conditions: 300°C, split ratio: 100/1
Injection Amount: 1 pL
Detection conditions: FID System, 300°C
Column temperature conditions: Starting at 80°C, the temperature was raised to 300°C at a rate of 10°C/min, and then maintained at 300°C for 10 minutes.

### [Production Example 1]

### Production of 1-(2-t-butylphenyloxy)-2-butanol (3)

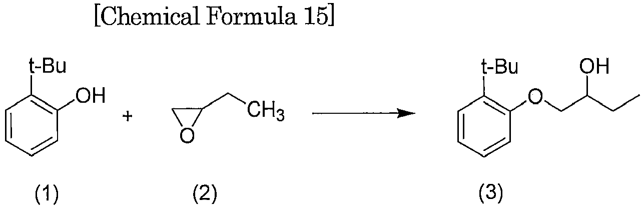

2-t-Butylphenol (1) (manufactured by FUJIFILM Wako Pure Chemical Corporation, 350 g) and 35 g of 48% sodium hydroxide aqueous solution (manufactured by Kanto Chemical Co., Inc.) were added to a 1-liter round-bottom flask provided with a Dimroth condenser and a dropping funnel in a nitrogen stream, and the resulting mixture was heated to 80°C. 1,2-Butylene oxide (2) (manufactured by Tokyo Chemical Industry Co., Ltd., 176 g) was added dropwise thereto over about 2 hours, followed by stirring at 80°C for 5 hours. After cooling the reaction mixture to room temperature (25°C), an organic layer was separated from the underlying sodium hydroxide aqueous solution and then distilled, whereby 1-(2-t-butylphenyloxy)-2-butanol (3) was obtained in a yield of 96%.

### [Production Example 2]

### Production of 1-(2-t-butylcyclohexyloxy)-2-butanol (4)

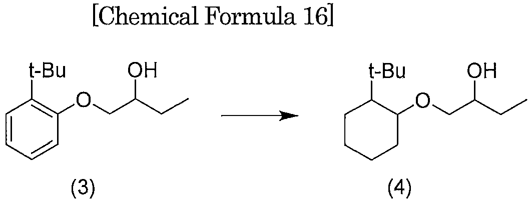

To a 500 ml autoclave, 1-(2-t-butylphenyloxy)-2-butanol (3) (250 g) obtained in Production Example 1, an activated carbon-supported palladium catalyst (manufactured by N.E. Chemcat Corporation, trade name: S-type, 50% water-containing product, palladium loading: 2%, 4.75 g), and an activated carbon-supported ruthenium catalyst (manufactured by N.E. Chemcat Corporation, 50% water-containing product, ruthenium loading: 5%, 0.25 g) were added, and the resulting mixture was reacted for 6 hours at 190°C and at a hydrogen pressure of 2.0 MPa.

After completion of the reaction, the catalysts were filtered off and the filtrate was distilled, whereby 1-(2-t-butylcyclohexyloxy)-2-butanol (4) having a purity of 100% was obtained in a yield of 70%. Analysis of the product by gas chromatography showed that the mass ratio of the cis isomer to the trans isomer was cis:trans = 57:43.

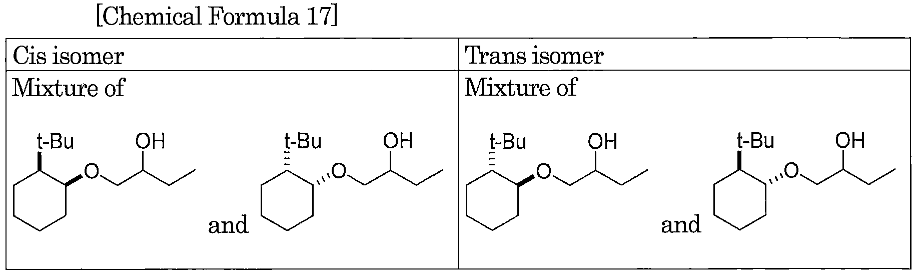

### [Production Example 3]

### Production of 1-(2-t-butylcyclohexyloxy)-butan-2-one (5)

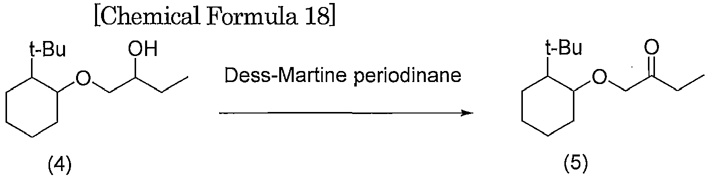

1-(2-t-Butylcyclohexyloxy)-2-butanol (4) (2 g) obtained in Production Example 2 was dissolved in dichloromethane (35.0 mL), and Dess-Martine periodinane (4.5 g) was then added at 0°C. After 10 minutes, the cooling bath was removed, and the reaction mixture was warmed to room temperature (25°C) and stirred for 30 minutes to carry out the reaction. As a post-treatment, a saturated aqueous sodium thiosulfate solution was added to quench the reaction. The mixture was extracted with diethyl ether, dried over sodium sulfate, and filtered. The solvent was then distilled off to obtain 1-(2-t-butylcyclohexyloxy)butan-2-one (5). The resulting crude product was purified by silica gel column chromatography to obtain 1-(2-t-butylcyclohexyloxy)butan-2-one (5) having a purity of 100% in a yield of 92%.

### [Example 1]

A fragrance composition was obtained by adding 9.981 g of 1-(2-t-butylcyclohexyloxy)-2-butanol (4) obtained in Production Example 2 to 19 mg of 1-(2-t-butylcyclohexyloxy)butan-2-one (5) obtained in Production Example 3 and mixing them (GC composition ratio of 1-(2-t-butylcyclohexyloxy)butan-2-one (5)/1-(2-t-butylcyclohexyloxy)-2-butanol (4) in the mixture = 0.19/99.81).

### [Example 2]

A fragrance composition was obtained by adding 9.979 g of 1-(2-t-butylcyclohexyloxy)-2-butanol (4) (cis/trans mass ratio = 57:43) obtained in Production Example 2 to 21 mg of 1-(2-t-butylcyclohexyloxy)butan-2-one (5) obtained in Production Example 3 and mixing them (GC composition ratio of 1-(2-t-butylcyclohexyloxy)butan-2-one (5)/1-(2-t-butylcyclohexyloxy)-2-butanol (4) in the mixture = 0.21/99.79).

### [Example 3]

A fragrance composition was obtained by adding 9.971 g of 1-(2-t-butylcyclohexyloxy)-2-butanol (4) obtained in Production Example 2 to 29 mg of 1-(2-t-butylcyclohexyloxy)butan-2-one (5) obtained in Production Example 3 and mixing them (GC composition ratio of 1-(2-t-butylcyclohexyloxy)butan-2-one (5)/1-(2-t-butylcyclohexyloxy)-2-butanol (4) in the mixture = 0.29/99.71).

### [Example 4]

A fragrance composition was obtained by adding 9.888 g of 1-(2-t-butylcyclohexyloxy)-2-butanol (4) obtained in Production Example 2 to 0.112 g of 1-(2-t-butylcyclohexyloxy)butan-2-one (5) obtained in Production Example 3 and mixing them (GC composition ratio of 1-(2-t-butylcyclohexyloxy)butan-2-one (5)/1-(2-t-butylcyclohexyloxy)-2-butanol (4) in the mixture = 1.12/98.88).

### [Example 5]

A fragrance composition was obtained by adding 0.97 g of 1-(2-t-butylcyclohexyloxy)-2-butanol (4) obtained in Production Example 2 to 30 mg of 1-(2-t-butylcyclohexyloxy)butan-2-one (5) obtained in Production Example 3 and mixing them (GC composition ratio of 1-(2-t-butylcyclohexyloxy)butan-2-one (5)/1-(2-t-butylcyclohexyloxy)-2-butanol (4) in the mixture = 3.00/97.00).

### [Comparative Example 1]

A fragrance composition was obtained by adding 0.9515 g of 1-(2-t-butylcyclohexyloxy)-2-butanol (4) obtained in Production Example 2 to 48.5 mg of 1-(2-t-butylcyclohexyloxy)butan-2-one (5) obtained in Production Example 3 and mixing them (GC composition ratio of 1-(2-t-butylcyclohexyloxy)butan-2-one (5)/1-(2-t-butylcyclohexyloxy)-2-butanol (4) in the mixture = 4.85/95.15).

### [Sensory Evaluation]

The odor of each of the fragrance compositions obtained in Examples 1 to 5 and Comparative Example 1 was evaluated by three expert panelists. The evaluation was made using a fragrance test paper (Daimonji Paper Co., Ltd., 150 mm × 7 mm). The fragrance compositions of Examples 1 to 5 and Comparative Example 1, which differ from each other in the ratio of the isomers, were each applied to the tip of the fragrance test paper, and the evaluation was performed indoors. Sensory evaluation was made in terms of odor characteristics and odor intensity. The odor characteristics and odor intensity were relatively evaluated on a scale of 1 to 5, with higher numbers representing better results. Odor persistence was evaluated by determining how many hours the odor lasted and by relatively rating the elapsed time and the odor intensity at each elapsed time point on a scale of 1 to 10, with higher numbers representing better results. In the sensory evaluation, the three expert panelists also described how they had felt about the quality and characteristics of the overall odor. The results obtained are shown in Tables 3 and 4.

A comprehensive evaluation of the usefulness of the odor of each fragrance composition as a fragrance material was conducted from the viewpoints of odor characteristics and odor intensity, and each fragrance composition was ranked according to the following criteria.
A: Highly interesting and of high value as a fragrance material
B: Having sufficient value as a fragrance material
C: Having almost sufficient value as a fragrance material
D: Having somewhat low value as a fragrance material

**[Table 3]**

| | Compound (5) ^{*1)} | Compound (4) *2) | Amber-like and woody characteristics | Odor sweetness | Odor volume | Persistence (hours) | | | | | Comprehensive evaluation |
|---|---|---|---|---|---|---|---|---|---|---|---|
| | | | | | | 0 | 8 | 24 | 48 | 72 | |
| Ex. 1 | 0.19 | 99.81 | 10 | 2 | 2 | 6 | 6 | 6 | 5 | 4 | C |
| Ex. 2 | 0.21 | 99.79 | 10 | 5 | 5 | 8 | 7 | 6 | 6 | 5 | B |
| Ex. 3 | 0.29 | 99.71 | 10 | 10 | 10 | 10 | 9 | 8 | 7 | 6 | A |
| Ex. 4 | 1.12 | 98.88 | 10 | 10 | 10 | 10 | 9 | 8 | 7 | 6 | A |
| Ex. 5 | 3.00 | 97.00 | 5 | 10 | 5 | 10 | 9 | 8 | 7 | 6 | B |
| Com. Ex. 1 | 4.85 | 95.15 | 1 | 10 | 1 | 10 | 9 | 8 | 7 | 6 | D |

| | | | | | | | | | | | |
|---|---|---|---|---|---|---|---|---|---|---|---|
| *1) Compound (5): 1-(2-t-butylcyclohexyloxy)-butan-2-one (5) *2) Compound (4): 1-(2-t-butylcyclohexyloxy)-2-butanol (4) | | | | | | | | | | | |

**[Table 4]**

| | Evaluation of odor |
|---|---|
| Ex. 1 | Strong amber-like, woody, and camphor-like odor, together with slightly perceived orris-like sweetness and volume, resulting in good balance. |
| Ex. 2 | Strong amber-like, woody, and slightly camphor-like odor, with imparted orris-like sweetness and good odor volume, resulting in excellent balance. |
| Ex. 3 | Strong amber-like, woody, and slightly camphor-like odor, with imparted orris-like sweetness and good odor volume, resulting in particularly excellent balance. |
| Ex. 4 | Strong amber-like, woody, and slightly camphor-like odor, with imparted orris-like sweetness and good odor volume, resulting in particularly excellent balance. |
| Ex. 5 | Moderately strong amber-like and woody odor is perceived, together with well-perceived orris-like sweetness and good odor volume, resulting in excellent balance. |
| Com. Ex. 1 | The original amber-like and woody characteristics are weakly perceived. Although the orris-like sweetness is strongly perceived, the odor lacks volume, resulting in an unbalanced fragrance. |

### [Evaluation Results]

As shown in Tables 3 and 4, the fragrance composition of the present invention is a fragrance composition having excellent balance, with orris-like sweetness and volume imparted to its original amber-like, woody (and camphor-like) odor.

## Claims

1. A fragrance composition comprising a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less: 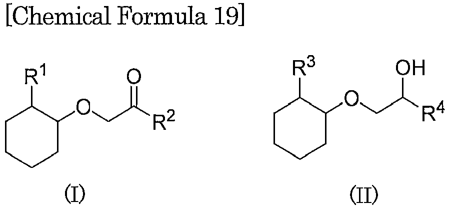 where
R¹ and R³ are each independently an alkyl group having 1 or more and 5 or less carbon atoms, and
R² and R⁴ are each independently a hydrogen atom or an alkyl group having 1 or more and 4 or less carbon atoms.

2. The fragrance composition according to claim 1, wherein R¹ and R³ are t-butyl groups.

3. The fragrance composition according to claim 1 or 2, wherein R² and R⁴ are independently a methyl group or an ethyl group.

4. The fragrance composition according to any one of claims 1 to 3, wherein R¹ and R³ are t-butyl groups, and R² and R⁴ are ethyl groups.

5. The fragrance composition according to any one of claims 1 to 4, wherein the mass ratio of the compound represented by the formula (I) to the compound represented by the formula (II) [compound represented by formula (I):compound represented by formula (II)] is 0.25:99.75 or more and 2.00:98:00 or less.

6. The fragrance composition according to any one of claims 1 to 5, further comprising at least one component that is other than the compounds represented by the formulae (I) and (II) and is selected from alcohols, phenols, esters, carbonates, aldehydes, ketones, acetals, ethers, lactones, and nitriles.

7. A cosmetic comprising the fragrance composition according to any one of claims 1 to 6.

8. A cleaning composition comprising the fragrance composition according to any one of claims 1 to 6.

9. A fiber treating agent composition comprising the fragrance composition according to any one of claims 1 to 6.

10. A method of using a composition as a fragrance-imparting component, the composition consisting of a compound represented by a general formula (I) and a compound represented by a general formula (II),
wherein a mass ratio of the compound represented by the formula (I) to the compound represented by the formula (II) [compound represented by formula (I):compound represented by formula (II)] is 0.10:99.90 or more and 4.00:96.00 or less: 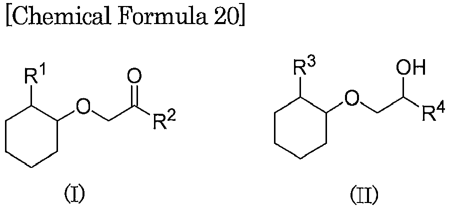 where
R¹ and R³ are each independently an alkyl group having 1 or more and 5 or less carbon atoms, and
R² and R⁴ are each independently a hydrogen atom or an alkyl group having 1 or more and 4 or less carbon atoms.
